# EUROPEAN PATENT SPECIFICATION

(11) **EP 2 768 523 B1**
(45) Date of publication and mention of the grant of the patent: **22.05.2019**
(21) Application number: 12842537.8
(22) Date of filing: 19.10.2012
(51) Int. Cl.: A61K 38/48, A61Q 19/06, A61K 9/00

(54) **METHOD OF TREATING OR REDUCING EFP**
VERFAHREN ZUR BEHANDLUNG ODER LINDERUNG VON VERSTOPFUNGEN ENTEROKUTANER FISTELN
PROCÉDÉ DE TRAITEMENT OU DE RÉDUCTION DE L'EFP

(30) Priority: 21.10.2011 US 201161549863 P
(43) Date of publication of application: 27.08.2014
(73) Proprietor: Endo Global Ventures, Hamilton HM12 (BM)
(72) Inventor: HART, Susan G., Emeigh, Oxford, PA 19363 (US)
(74) Representative: Lee, Nicholas John
(86) International application number: PCT/US2012/061063
(87) International publication number: WO 2013/059619

(56) References cited:
- US-A- 4 645 668
- US-A1- 2007 224 184
- US-A1- 2011 243 909
- US-B2- 7 622 130
- US-B2- 7 824 673
- Auxilium Pharmaceuticals ET AL: "Press Release", , 26 January 2011 (2011-01-26), XP55403183, Retrieved from the Internet: URL:https://www.sec.gov/Archives/edgar/dat a/1182129/000119312512024205/d290313dex991 .htm [retrieved on 2017-09-01]

## Description

### BACKGROUND OF THE INVENTION

Edematous fibrosclerotic panniculopathy (EFP), more commonly referred to as cellulite, is a condition that presents as a topographical alteration in the appearance of the skin and affects about 90% of postpubertal women (Rawlings (2006), Int J Cosmetic Sci 2006; 28: 175-90; Khan et al. (2010), J Am Acad Dermatol 2010; 62: 361-70). EFP appears as dimpled skin and gives the skin what is commonly described as an orange-peel appearance. The dermal septae, composed of collagenase, are believed to play a causative role in the dimpling of the skin.

Collagenase, an enzyme that has the specific ability to digest collagen, has been used to treat a variety of collagen-mediated diseases, including, for example, Dupuytren's contracture, Peyronie's disease, lipoma and adhesive capsulitis. U.S. Patent No. 4,645,668 and U.S. Patent App. Pub. No. 20070224184 also disclose certain uses of collagenase. A major source of collagenase is from the fermentation of the bacterium, *Clostridium histolyticum.* An injectable formulation comprising *C*. *histolyticum* collagenase I and collagenase II is sold under the trade name XIAFLEX® and is approved by the U.S. Food and Drug Administration (FDA) for the treatment of Dupuytren's contracture.

### SUMMARY OF THE INVENTION

The present invention is based on the discovery that one or more low dose injections of collagenase to an area affected by EFP are effective to reduce or treat such EFP. The amount of collagenase in a single injection may be as low as 5 ABC units, or 0.00029 mg (0.29 µg). The total dose of collagenase administered depends on the size of the treatment area, and thus may be between about 5 to about 5000 ABC units. The concentration of collagenase may be between about 50 to about 2000 ABC units/milliliter (ml). 10,000 ABC units is equivalent to 0.58 mg collagenase.

The invention is directed to a cosmetic method of treating or reducing EFP in a patient comprising the steps of: providing a pharmaceutical composition comprising a pharmaceutically acceptable carrier, collagenase I and collagenase II in a 1:1 mass ratio with a specific activity of 10,000 ABC units per 0.58 mg of collagenase, and administering by subdermal injection to said patient 5-200 ABC units per injection of the pharmaceutical composition to an area affected by EFP, wherein when multiple injections are administered, injection sites are about 1 to about 4 cm from one another. In certain aspects, a plurality of subdermal injections are administered at a plurality of injection sites. In additional embodiments, the concentration of collagenase administered is between about 50 to about 2000 ABC units per milliliter. In certain additional embodiments, each injection of collagenase is administered in a volume of about 0.5 ml or less. In a further aspect, the total dose of collagenase administered is between about 5 to about 2000 ABC units.

Disclosed herein is a method of treating or reducing EFP in a patient comprising administering to said patient one or a plurality of subdermal injections of collagenase within an area affected by EFP, wherein the total dose of collagenase administered to the affected area is between about 5 to about 5000 ABC units, and wherein the plurality of injections are administered at a plurality of injection sites. Each injection may be between about 5 to about 200 ABC units. The concentration of collagenase administered may be between about 50 to about 2000 ABC units per milliliter and/or each injection of collagenase may be administered in a volume of about 0.5 ml or less.

Also disclosed herein is a method of treating or reducing EFP in a patient comprising administering to said patient one or a plurality of subdermal injections of collagenase to an area affected by EFP, wherein the dose of collagenase administered per injection to the affected area is between about 5 to about 200 ABC units and wherein the concentration of collagenase administered to the affected area is between about 50 to about 2000 ABC units per milliliter.

Also disclosed herein is a method of treating or reducing EFP in a patient comprising administering one or a plurality of subdermal injections of collagenase to an area affected by EFP wherein the concentration of collagenase administered to the affected area is between about 50 to about 2000 ABC units per milliliter and wherein the volume of each injection of collagenase is about 0.5 ml or less.

In some embodiments, the dose of collagenase administered per injection is between about 5 to about 100 ABC units. In additional embodiments, the dose of collagenase administered per injection is between about 5 to about 50 ABC units.

The collagenase can be derived from a bacterial source or can be a recombinant form of collagenase. In some embodiments, the collagenase is purified from *Clostridium histolyticum.* The collagenase for use in the invention comprises collagenase I and collagenase II. In yet further embodiments, the collagenase is collagenase I and collagenase II purified from *Clostridium histolyticum* and comprises collagenase I and collagenase II.

### BRIEF DESCRIPTION OF THE DRAWINGS

The foregoing and other objects, features and advantages of the invention will be apparent from the following more particular description of preferred embodiments of the invention, as illustrated in the accompanying drawings in which like reference characters refer to the same parts throughout the different views. The drawings are not necessarily to scale, emphasis instead being placed upon illustrating the principles of the invention.
FIG. 1 is a depiction of the injection template where each dot within the octagon represents an injection site. The X within the circle represents the injection site to the "central dimple." The four corners are labeled as upper right (UR), lower right (LR), upper left (UL), and lower left (LL).
FIG. 2 is a depiction of buttock and posterior upper thigh with template corner markings.

### DETAILED DESCRIPTION OF THE INVENTION

A description of preferred embodiments of the invention follows.

As discussed above, the present invention is based on the discovery that low dose injections of collagenase to an area affected by EFP are effective to lyse collagen and thereby reduce or treat such EFP. The amount of collagenase in a single injection may be as low as 5 ABC units, or 0.00029 mg. The total dose of collagenase administered depends on the size of the treatment area, and is thus between about 5 to about 5000 ABC units and/or wherein the concentration of collagenase is between about 50 to about 2000 ABC units/milliliter (ml). The doses and concentrations of collagenase employed according to the method of the present invention are substantially lower than the doses and concentrations of collagenase currently used and approved by the U.S. FDA for the treatment of Dupuytren's contracture.

The words "a" or "an" are meant to encompass one or more, unless otherwise specified.

"Treating" or "treatment" of EFP includes the administration of the compositions or agents described herein to improve the appearance of the skin previously affected by EFP and/or to achieve an improved aesthetic outcome. Treatment of EFP can, for example, encompass a visual reduction in the severity of EFP or a visual reduction in the severity or number of skin dimples.

Disclosed herein is a method of treating or reducing EFP in a patient comprising administering one or a plurality subdermal injections of collagenase to an area affected by EFP, wherein the plurality of injections are injected at a plurality of injections sites within an area affected by EFP, wherein the dose of collagenase administered per injection is about 5 to about 200 ABC units. In additional embodiments, the dose of collagenase per injection is about 5 to about 100 ABC units, or about 5 to about 50 ABC units, or about 10 to about 100 ABC units, or about 10 to about 50 ABC units. In additional embodiments, the total dose of collagenase administered to the affected area is between about 5 to about 5000 ABC units. Disclosed herein is a method comprising administering collagenase to the area affected by EFP wherein the concentration of collagenase administered to the affected area is about 50 to about 2000 ABC units/ml. Also disclosed herein is a method of treating or reducing EFP in a patient comprising administering a plurality of injections of collagenase to an area affected by EFP, wherein the dose of collagenase per injection is between about 5 to about 200 ABC units.

An area affected by EFP (also referred to herein as the "target area" or "treatment area") is typically an area of the skin on the thighs and/or buttocks characterized by one or more dimples. In some embodiments, the area of EFP treated with the one or plurality of injections of collagenase is an area of the lateral upper aspect of the thigh or an area of the buttocks and does not involve the gluteal fold. The target area to be treated with collagenase can also be an area having a photonumeric EFP severity scale (CSS) score of ≥ 10 representing moderate to severe EFP severity within the right or left buttock or the right or left thigh (Hexsel et al. (2009). A validated photonumeric EFP severity scale. JEADV 2009; 23:523-52). The CSS is a photonumeric scale that looks at five key morphologic features of EFP: (A) number of depressions, (B) depth of depressions, (C) morphological appearance of skin surface, (D) laxity, flaccidity or sagging of skin, and (E) the classification scale originally described by Nümberger and Müller, J Dermatol Surg Oncol 1978; 4(3): 221-29.) (Hexsel *et al*., 2009). Each of these features is evaluated on a 4-point scale from a low of 0 to a high of 3. The scale ranges from 0 to 15.

The target area treated with the one or plurality of injections of EFP is generally an area of the skin characterized by one or more dimples that are visually evident when the patient is standing. The geometric area of the treatment area depends on the size of the area affected by EFP. Thus, for example, if the area affected by EFP includes one dimple, the geometric area of the target area can be about 1 cm² to about 5 cm². In additional aspects, the area affected by EFP comprises multiple dimples and has a geometric area greater than 1 cm². In one embodiment, the target area has a geometric area between about 1 cm² to about 200 cm². As will be understood, because the target area is generally an area of the thigh or buttocks, the area can, for example, be roughly rectangular in shape and have a length of about 1 to about 15 cm, and a width of about 1 to about 10 cm. In an additional example, the target area has a length of about 6 to about 15 cm and a width of about 4 to about 10 cm. The person of skill in the art will also understand that the target area can be roughly circular or any other geometric shape depending on the desired area for treatment. The target area can optionally be characterized by at least one skin dimple approximately at the center of the area of EFP treated, wherein the area of EFP is treated with the one or a plurality of injections. The sites for injection of collagenase can be numbered and spaced within the area affected by EFP such as to allow for even distribution and efficacy of the injected collagenase. The number of collagenase injections will generally depend on the size of the area to be treated. In some embodiments, the number of injection sites is at least 1 or more. In other aspects, the number of injection sites is at least about 3 or more. In other aspects, the number of injection sites is at least about 5 or more. In yet other aspects, the number of injection sites is at least about 7 or more. In a further aspect, the number of injection sites is at least 10 or more.

In one embodiment, in which the area to be treated is a single dimple area or otherwise having a geometric area of less than about 5 cm², there is only a single site of injection, and between about 5 and about 200 ABC units of collagenase is injected. Optionally, the single injection can be injected into the center of the dimple. In embodiments in which the treatment area is larger, there will be multiple sites of injection and between about 5 to about 200 ABC units of collagenase is administered per injection. Optionally, the plurality of injections include at least one injection administered to the center of one dimple. When multiple injections are administered in accordance with the invention, the sites of injection are about 1 to about 4 cm from one another. In yet other aspects, the sites of injection can be about 2 to about 3 cm from one another. For example, in certain embodiments, the area affected by EFP has a width about 8 cm and a length of about 10 cm in dimensions and the number of collagenase injections administered is 10 and the distance between injection sites is about 2.5 cm. In certain aspects, an injection template (for example, made of a thin clear material) which includes markings showing the intended injection sites is placed over the target area and the one or more injections are made at the sites indicated by the template.

The target area is treated with one or a plurality of concurrent injections of collagenase. As used herein, concurrent injections are injections administered at the same time or sequentially within the same period of time, i.e., during a single treatment session. As discussed above, the total dose of collagenase administered to the area affected by EFP may be between about 5 to about 5000 ABC units of collagenase. The total dose of collagenase administered depends on the size of the treatment area. The total dose of collagenase is the sum of the doses administered using one or a plurality of injections of collagenase. In certain aspects, each injection of collagenase will comprise equal doses of collagenase. For example, when the number of injections is 10, to be distributed over the treatment area, and each injection is 200 ABC units of collagenase, the total dose of collagenase to be administered to the target area will be 2000 ABC units. By way of further example, when the number of injections is 10 to be distributed over the treatment area, and each injection is 5 ABC units of collagenase, the total dose of collagenase to be administered to the target area will be 50 ABC units. By way of further example, when the number of injections is 5 to be distributed over the treatment area, and, each injection is 5 ABC units of collagenase, the total dose of collagenase to be administered to the target area will be 25 ABC units. When only a single injection is to be performed and the dose selected is 5 ABC units, the total dose of collagenase administered will be 5 ABC units. The concentration of collagenase administered to the area affected by collagenase is between about 50 ABC units/ml to about 2000 ABC units/ml. Collagenase can be administered in a volume per injection of about 0.5 ml or less. In another aspect, collagenase can be administered in a volume per injection of about 0.1 ml to about 0.5 ml. The total volume of collagenase injected can, for example, be between about 0.1 ml (when only a single injection site is used) to about 7 ml (for multiple sites of injections), and higher for larger size treatment areas. In one embodiment where the treatment area is about 80 cm², the total volume injected is about 1 to about 5 ml, summed from 10 injections of between 0.1 ml and 0.5 ml.

Collagenase is an enzyme that has the specific ability to digest collagen. One commercial source of collagenase is from fermentation by *Clostridium histolyticum.* In certain aspects, the collagenase comprises a combination of purified *Clostridium histolyticum* collagenase I and collagenase II. Collagenase I and collagenase II are present in the pharmaceutical composition for use in the invention in a mass ratio of 1 to 1. Collagenase AUX I has a single polypeptide chain consisting of approximately 1000 amino acids with a molecular weight of 115 kDa. Collagenase AUX II also has a single polypeptide chain consisting of about 1000 amino acids with a molecular weight of 110 kDa. Crude collagenase obtained from *C*. *histolyticum* can be purified by a variety of methods known to those skilled in the art, including, for example, heparin affinity chromatography, ammonium sulfate precipitation, hydroxylapatite chromatography, size exclusion chromatography, ion exchange chromatography, and metal chelation chromatography. Methods of purification of crude collagenase obtained from *C*. *histolyticum* are also described in U.S. Pat. No. 7,811,560. As discussed above, an injectable formulation comprising *C*. *histolyticum* collagenase I and collagenase II is sold in the U.S. under the trade name XIAFLEX® and is approved by the U.S. FDA for the treatment of Dupuytren's contracture. The collagenase can, for example, be a parenteral lyophilized product comprised of two collagenases in an approximate 1:1 mass ratio, Collagenase I (AUX-I, Clostridial type I collagenase) and Collagenase II (AUX-II; Clostridial type II collagenase). Preferably, the collagenase comprises collagenase I and collagenase II having a mass ratio of about 1 to 1 and having a purity of at least about 95% by area as determined by reverse phase high performance liquid chromatography (as described, for example, in U.S. Pat. No. 7,811,560).

Collagenase compositions of the invention can also be prepared by mixing either a specific number of activity units or specific masses of the purified enzymes. Collagenase activity can be measured by the enzyme's ability to hydrolyze either synthetic peptide or collagen substrate. Those skilled in the art will recognize that enzyme assays other than those disclosed herein may also be used to define and prepare functionally equivalent enzyme compositions.

It is understood that the terms "Collagenase I", "ABC I", "AUX I", "collagenase AUX I", and "collagenase ABC I" mean the same and can be used interchangeably. Similarly, the terms "Collagenase II", "ABC II", "AUX II", "collagenase AUX II", and "collagenase ABC II" refer to the same enzyme and can also be used interchangeably.

In certain additional embodiments, the collagenase administered according to a method described herein is a recombinant collagenase.

The collagenase is administered in a pharmaceutical composition comprising collagenase I and collagenase II in a 1:1 mass ratio with a specific activity of 10,000 ABC units per 0.58 mg of collagenase and a pharmaceutically acceptable carrier. In some embodiments, the composition does not include a protease enzyme other than collagenase (other than a small or trace amount of proteolytic enzyme that may be present in the collagenase purified from a bacterial fermentation). In additional embodiments, the pharmaceutical composition does not include an enzyme other than collagenase (other than a small or trace amount of enzyme that may be present in the collagenase purified from a bacterial fermentation). The person of skill in the art will understand that a collagenase derived from a bacterial fermentation, even after purification, may contain small or trace amounts of impurities, including other enzymes, such as other protease enzymes. The small or trace amount of impurity can, for example, be less than about 5%, less than about 4%, less than about 3%, less than about 2% or less than about 1% of the collagenase composition. In some embodiments, the small or trace amount of impurity can be less than about 1%, 2%, 3%, 4% or 5% by area, as determined by reverse phase high performance liquid chromatography. In another aspect, the pharmaceutical composition consists essentially of collagenase and a pharmaceutically acceptable carrier or diluent. As used herein, a pharmaceutical composition "consisting essentially of collagenase and a pharmaceutically acceptable carrier or diluent" is intended to exclude from the pharmaceutical compositions other protease enzymes and hyaluronidase other than small or trace amounts as described above. In certain additional aspects, a composition "consisting essentially of collagenase" excludes from the compositions an enzyme other than collagenase. In yet another embodiment, the pharmaceutical composition consists of collagenase and a pharmaceutically acceptable salt thereof.

The method of treating or reducing EFP can further include the pre-treatment of the target area with a local anaesthetic agent.

Disclosed herein is the treatment or reduction of EFP in a patient consisting essentially of administering one or a plurality injections of collagenase to an area affected by EFP wherein the dose of collagenase administered is between about 5 to about 200 ABC units and optionally, further wherein the concentration of collagenase is about 50 to about 2000 ABC units/ml. Also disclosed herein is the treatment or reduction of EFP in a patient consisting of administering a plurality of injections of collagenase wherein the dose of collagenase administered per injection is between about 5 to about 200 ABC units and optionally, further wherein the concentration of collagenase is about 50 to about 2000 ABC units/ml.

The pharmaceutically acceptable carrier can be one or more liquid carriers or excipients appropriate for injection. As used herein, the term "pharmaceutically acceptable carrier or excipient" means a non-toxic, inert, liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; glycols such as propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, perfuming agents, preservatives and antioxidants can also be present in the composition, according to the judgment of the formulator. Injectable preparations, for example, sterile injectable aqueous or oleaginous suspensions, may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1, 3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use. The sterile solutions can also be lyophilized for later use. In one embodiment, the pharmaceutical composition comprising collagenase is a lyophilized injectable composition formulated with lactose. In one embodiment each milligram of injectable collagenase is formulated with 1.9 mg of lactose. In another embodiment, each milligram of injection collagenase preferably has approximately 2800 SRC units and 51000 units measured with a potency assay using a synthetic substrate, pzGPGGPA.

In another embodiment, the collagenase composition used according to a method of the invention is a lyophilized injectable composition formulated with sucrose, Tris at a pH level of about 8.0. For example, 1.0 mg of the drug substance of the invention is formulated in 60 mM sucrose, 10 mM Tris, at a pH of about 8.0 (this equates to 20.5 mg/mL of sucrose and 1.21 mg/mL of Tris in the formulation buffer). Generally, a source of calcium is included in the formulation, such as calcium chloride.

The invention will be better understood in connection with the following examples, which are intended as an illustration only and not limiting of the scope of the invention. Various changes and modifications to the disclosed embodiments will be apparent to those skilled in the art and such changes and modifications may be made without departing from the scope of the appended claims.

### EXAMPLES

### Example 1: Effectiveness of Collagenase Clostridium Histolyticum in lysing dermal collagen in Gottingen minipigs

Collagenase Clostridium Histolyticum (Auxilium Product Operation, Malvern, PA) is a parenteral lyophilized product comprised of two collagenases in an approximate 1:1 mass ratio, Collagenase I (AUX-I, Clostridial type I collagenase) and Collagenase II (AUX-II; Clostridial type II collagenase). These collagenases are isolated and purified from the fermentation of *Clostridium histolyticum.* The vehicle for reconstitution of the lyophilized product was saline (0.9% sodium chloride) with 0.03% (2 mM) calcium chloride. The vehicle for dilution and preparation of the formulations described in more detail below was saline (0.9% sodium chloride for injection; Baxter Healthcare Corporation, Deerfield, IL) with 10 mM (0.13%) TRIZMA ® (Sigma Aldrich, Inc., St. Louis, MO), 60 mM (2.0%) sucrose (Sigma Aldrich, Inc., St. Louis, MO), and 2 mM calcium chloride (pH 8.0) (Aldrich Chemical Corporation, Allentown, PA). IN HCl (hydrochloric acid) was used to bring the pH of the vehicle for dilution to 8.0. The vehicle for dilution was mixed throughout preparation, sampling and dose administration procedures. The vehicle for dilution was then sterile-filtered using a 0.22-µm PVDF syringe filter into a sterile vial and capped with a septum. The vehicle for dilution was prepared using aseptic technique within a laminar flow hood using sterilized glassware and utensils.

Dosing formulations were prepared at the test article (Collagenase Clostridium Histolyticum) concentrations indicated in the following table:

**Table 1**

| Treatment Number | Test Article Concentration ^{a} (mg/mL)/(ABC units/ml) | pH ^{b} |
|---|---|---|
| 1 | 0.0015/25.9 | 8 |
| 2 | 0.003/51.2 | 8 |
| 3 | 0.005/86.2 | 8 |
| 4 | 0.009/ 55.2 | 8 |
| 5-7 | 0.015/258.6 | 8 |
| 8-9 | 0.03/517.2 | 8 |
| 10 | 0.07/1206.9 | 8 |
| 11 | 0.09/1551.7 | 8 |
| 12 | 0.15/2586.2 | 8 |

| | | |
|---|---|---|
| ^{a} = The test article formulations were not adjusted for purity. ^{b} = pH measurement using litmus paper. | | |

The dosing formulations were prepared on the day of dosing. The lyophilized product was reconstituted using the vehicle for reconstitution to obtain a 2.5 mg/mL stock test article solution. The vehicle for dilution was used to dilute the stock test article solution and prepare the formulations to be used for dosing. The formulations were maintained on wet ice prior to, and during dosing. Formulations were prepared using aseptic technique within a laminar flow hood using sterilized glassware and utensils.

Göttingen minipigs were used as the test system on this study. This species and breed of animal is recognized as appropriate for general toxicity studies and has been utilized as a biomedical research model in a wide variety of disciplines. Swine exhibit many similarities with man in cardiovascular anatomy and physiology, digestive physiology, and integumentary structure and function. Because of the similarity of the anatomy and physiology of the skin between swine and man, swine have become standard models for plastic surgery, wound healing, and dermal toxicity studies (Svendsen et al, (1998), Scandinavian Journal of Laboratory Animal Science 1998, 25 (supplement 1):27-30). Historically, the swine has been used in toxicological assessment of prospective therapeutics.

Göttingen minipigs (4 males and 3 females) were received in good health from Marshall BioResources, North Rose, NY. The animals were approximately 3 to 4 months old at receipt. Each animal received one dose of all 12 treatments, for a total dose per animal of approximately 0.43 mg Collagenase Clostridium Histolyticum. The animals were approximately 4 months old at dose administration. Body weights ranged from 7185 g to 8646 g for the males and 9333 g to 9633 g for the females at dosing. Test article formulations were administered to animals anesthetized by telazol/xylazine on study day 0 by subcutaneous injection (bolus). The day prior to injection, the designated areas for injection were clipped if necessary. Six injections were administered along the right and left lateral trunk of each animal using an appropriately sized syringe and needle (16-23 gauge). Animals were not dosed in the same site more than once. Each subcutaneous injection consisted of a different, randomly assigned treatment (combination of formulation concentration and dose volume). Injection sites were spaced sufficiently apart to prevent possible reactions from adjacent injection sites. The area immediately surrounding the site of test article deposition was marked using permanent marker and remarked as needed. Individual injection sites were labeled. The selected route of administration for this study was subcutaneous injection.

No drug-related histologic effects were detected in adipocytes, nerves, adnexal structures (hair follicles, sweat glands and sebaceous glands) or the overlying epidermis. There were no consistent differences in collagen lysis between the sexes or between the animals euthanized at 24 or 48 hours post-dosing in the estimated extent of collagen lysis and the results are combined by gender for the 24 and 48 hour post-dosing necropsies in the table below. At concentrations less than 0.015 mg/mL, the extent of collagen lysis was generally dose responsive. At ≥ 0.015 mg/mL the extent of collagen lysis was generally proportional to the dose volume injected as opposed to the total dose or formulation concentration (for example, test articles 7 and 8 represent the same dose given at a lower concentration and higher volume for test article 7).

**Table 2**

| **Test Article** | **Concentration (mg/mL)/(ABC units/ml** | **Dose Volume (mL)** | **Total Dose (mg)/ABC units** | **Mean Extent of Collagen Lysis (cm²)^{a}** | | |
|---|---|---|---|---|---|---|
| | | | | **Males** | **Females** | **Both** |
| 1 | 0.0015/25.9 | 0.2 | 0.0003/5.17 | 0.38 | 2.90 | 1.64 |
| 2 | 0.003/51.2 | 0.2 | 0.0006/10.3 | 2.15 | 1.77 | 1.96 |
| 3 | 0.005/86.2 | 0.2 | 0.001/17.2 | 1.81 | 1.84 | 1.82 |
| 4 | 0.009/155.2 | 0.2 | 0.0018/31.0 | 3.13 | 2.27 | 2.70 |
| 5 | 0.015/258.6 | 0.05 | 0.00075/12.9 | 1.89 | 3.04 | 2.47 |
| 6 | 0.015/258.6 | 0.1 | 0.0015/25.9 | 4.15 | 2.82 | 3.48 |
| 7 | 0.015/258.6 | 0.2 | 0.003/51.2 | 5.84 | 3.89 | 4.87 |
| 8 | 0.03/517.2 | 0.1 | 0.003/51.2 | 3.93 | 3.57 | 3.75 |
| | | | | | | |
| 9 | 0.03/517.2 | 0.2 | 0.006/103.4 | 6.05 | 4.08 | 5.07 |
| 10 | 0.07/1206.9 | 0.1 | 0.007/120.7 | 6.37 | 4.78 | 5.57 |
| 11 | 0.09/1551.7 | 0.1 | 0.009/155.2 | 5.50 | 4.60 | 5.05 |
| 12 | 0.15/2586.2 | 0.1 | 0.015/258.6 | 5.72 | 4.43 | 5.07 |

| | | | | | | |
|---|---|---|---|---|---|---|
| a = For each gender, the mean extent of collagen lysis was combined for all three animals at the 24 and 48 hour necropsy intervals. | | | | | | |

Collagenase Clostridium Histolyticum administered as single subcutaneous injections at concentrations ranging from 0.0015 to 0.15 mg/mL resulted in local swelling at sites treated with Collagenase Clostridium Histolyticum concentrations ≥0.015 mg/mL and collagen lysis (the expected pharmacologic effect of Collagenase Clostridium Histolyticum) at all doses, dose volumes, and formulation concentrations in this study. The minimally effective dose in this study was 0.0003 mg protein and the minimally effective concentration was 0.0015 mg/mL. At concentrations less than 0.015 mg/mL, a dose response generally was apparent for the extent of collagen lysis. At concentrations ≥0.015 mg/mL, the extent of effective collagen lysis reflected the dose volume given more than the total dose or formulation concentration.

Collagen lysis was accompanied consistently by a number of secondary changes. These changes included the following: hemorrhage and/or acute inflammation seen consistently at all effective dose levels; necrosis of the muscle fibers of the panniculus carnosis at ≥0.003 mg/mL (total doses ≥ 0.0006 mg protein); perivascular and intramural edema, neovascularization/fibrosis, vascular necrosis, and/or thrombosis seen sporadically at concentrations ≥0.009 mg/mL (total doses ≥0.0018 mg protein); and arterial intramural hemorrhage seen sporadically at formulation concentrations ≥0.030 mg/mL (total doses ≥0.003 mg protein). There were no Collagenase Clostridium Histolyticum-mediated effects on adipocytes, nerves, adnexal structures or the overlying epidermis.

Results from this study showed some degree of histologically detectable collagen lysis of the dermal septa at all Collagenase Clostridium Histolyticum dose concentrations; however, more areas of complete lysis of dermal septa were seen at concentrations ≥0.015 mg/mL. In this study, there were no Collagenase Clostridium Histolyticum mediated effects on adipocytes, nerves, adnexal skin structures or the overlying epidermis. Local effects of Collagenase Clostridium Histolyticum (hemorrhage and/or acute inflammation) at each concentration were observed upon gross and/or microscopic examination at necropsy. There were no consistent differences in collagen lysis between the sexes or between the animals euthanized at 24 or 48 hours post-dosing in the estimated extent of collagen lysis.

At concentrations effective in the complete lysis of the dermal septae, dose and volume-dependent collagen lysis was noted to occur within a diameter of approximately 2.5 cm in the minipig study.

### Example 2: Phase 1b, open-label, dose-escalation and pharmacokinetic study for the treatment of EFP

The objectives of this study are to assess the safety, effectiveness, pharmacokinetics, and immunogenicity of Collagenase Clostridium Histolyticum at increasing total doses in an 80 cm² area, ranging from 0.0029 mg to 0.116 mg (50 to 2000 ABC units) and increasing concentrations ranging from 0.0029 mg/mL to 0.116 mg/mL (50 to 2000 ABC units per mL) in the treatment of adult women with EFP.

Based on the findings discussed above in the minipig study (Example 1), the injection template for the Phase 1 clinical study is designed to allow for a distance of approximately 2.5 cm between injections. This distance is expected to enable adequate distribution and activity of Collagenase Clostridium Histolyticum within the 8 cm x 10 cm area of EFP without significant gaps or overlap within the treatment area. In an attempt to determine the optimal dose for the treatment of EFP (for example, efficacious with minimal local adverse effects), dose selection was made following consideration of the favorable safety profile of Collagenase Clostridium Histolyticum to date both in clinical development and clinical practice as well as on data from the minipig study. As a result, a starting dose of 0.0029 mg injected as 10 separate 0.00029 mg doses within the 8 cm x 10 cm target EFP area at a concentration of 0.0029 mg/mL is proposed for the Phase 1 study.

This study is a Phase 1b, open-label, dose-escalation and pharmacokinetic study. During the screening visit, while the subject is standing, the investigator or qualified designee will examine the left and right buttock and the left and right thigh and select a quadrant that has a photonumeric EFP severity scale (CSS) score of ≥ 10 representing moderate to severe EFP severity (Hexsel et al., 2009). The investigator or qualified designee will identify an area of EFP within the selected quadrant that is at least 8 cm x 10 cm (i.e., target EFP area) and is suitable for treatment (i.e., that is on the lateral upper aspect of the thigh or within the buttock and does not involve the gluteal fold). The target EFP area must be evident when the subject is standing, without the use of any manipulation such as skin pinching or muscle contraction. Each subject will be screened for study eligibility within 21 days before injection of study drug on Day 1.

In this study, 63 subjects will each receive 10 concurrent equal volume injections of Collagenase Clostridium Histolyticum within the target EFP area. Following screening and determination of study eligibility, subjects will be assigned sequentially to Cohorts 1, 2, 3, and 4 (Table 3). The first nine subjects will be assigned to Cohort 1 (1 mL total volume/target EFP area). Within each of following Cohorts 2 to 4, the first nine subjects in each cohort will be assigned to the 5 mL total volume/target EFP area followed by the next nine subjects who will be assigned to the 1 mL total volume/target EFP area. Dosing will start with Cohort 1. Dosing in Cohorts 2 and 3 will not begin until the safety of subjects in Cohort 1 has been evaluated by the safety monitoring committee (SMC). Dosing in Cohort 4 will not begin until the safety of subjects in Cohorts 2 and 3 has been evaluated by the SMC.

The doses proposed to treat an 8 cm x 10 cm area of EFP represent between 0.5% (0.0029 mg) and 20% (0.116 mg) of the dose used in a single injection for Dupuytren's contracture at a concentration that is between 0.1% (0.0029 mg/mL) and 5% (0.116 mg/mL) of the concentration used in the approved product for Dupuytren's contracture.

**Table 3: Study Drug Assignment by Cohort**

| **Cohort** | **Dose CCH/Target EFP Area^{a}** | **Volume/Target EFP Area^{a}** | **Concentration** | **EFP Dose/Target EFP Area as a Percent of the Approved Dupuytren's Dose (0.58 mg)** | **EFP Concentration as a Percent of the Approved Dupuytren's Concentration (2.3 mg/mL)** |
|---|---|---|---|---|---|
| 1 | 0.0029 mg (50 U)^{b} | 1 mL | 0.0029 mg/mL (50 U/mL) | 0.5% | 0.1% |
| 2a | 0.0145 mg (250 U) | 5 mL | 0.0029 mg/mL (50 U/mL) | 2.5% | 0.1% |
| 2b | 0.0145 mg (250 U) | 1 mL | 0.0145 mg/mL (250 U/mL) | 2.5% | 0.6% |
| 3a | 0.0435 mg (750 U) | 5 mL | 0.0087 mg/mL (150 U/mL) | 7.5% | 0.4% |
| 3b | 0.0435 mg (750 U) | 1 mL | 0.0435 mg/mL (750 U/mL) | 7.5% | 1.9% |
| 4a | 0.116 mg (2000 U) | 5 mL | 0.0232 mg/mL (400 U/mL) | 20% | 1% |
| 4b | 0.116 mg (2000 U) | 1 mL | 0.116 mg/mL (2000 U/mL) | 20% | 5% |
| ^{a} A total of 10 single injections at the target injection sites across the 8 cm x 10 cm target EFP area. CCH is Collagenase Clostridium histolyticum | | | | | |
| ^{b} U is ABC units | | | | | |

The components of Collagenase Clostridium Histolyticum are: mixed collagenase AUX-I and AUX-II, 10 mM tris, 60 mM sucrose. The components of Collagenase Clostridium Histolyticum sterile Diluent A for reconstitution are: 0.03% (2 mM) calcium chloride (CaCL) in 0.9% (154 mM) sodium chloride (NaCL) solution, pH 6.0-7.0, is supplied as a terminally-sterilized liquid at 3.0 mL per vial. The components of sterile Collagenase Clostridium Histolyticum buffered sterile Diluent B for further dilution are: 0.03% (2 mM) calcium chloride and 0.12% (10 mM) tromethamine (TRIS) in 0.9% (154 mM) sodium chloride, pH 8.0 chloride is supplied as a terminally-sterilized liquid at 3.0 mL per vial. After reconstitution with Diluent A and further dilution with Diluent B, the A4500 solution can be kept at room temperature (20° to 25°C/68° to 77°F) for up to one hour or refrigerated at 2° to 8°C (36° to 46°F) for up to 4 hours prior to administration.

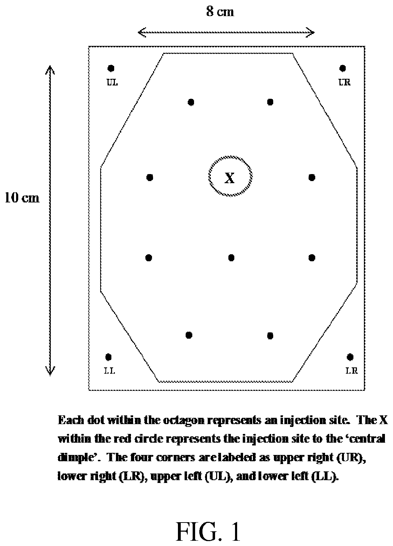

An injection template will be made of a thin clear material. The 8 cm x 10 cm injection template will be pre-stamped with the diagram and injection sites shown in FIG. 1. Each of the black dots within the octagon represents an injection site. The encircled 'X' represents the injection site of the central dimple (i.e., the area of the deepest depression in the target area (FIG. 1). Each black dot (including the site of the central dimple and the four corners) will be cut-out to enable the investigator to mark the target EFP area as needed. Each dot within the octagon shown in FIG. 1 represents an injection site. The X within the circle represents the injection site to the 'central dimple'. The four corners are labeled as upper right (UR), lower right (LR), upper left (UL), and lower left (LL).

During the screening visit, while the subject is standing, the investigator or qualified designee will examine the left and right buttock and the left and right posterior upper thigh and select a quadrant that has moderate to severe EFP (CSS score of ≥10). The investigator or qualified designee will identify an area of EFP within the selected quadrant that is at least 8 cm x 10 cm (referred to in this example as the target EFP area) and is suitable for treatment (for example, is within posterior upper thigh or within the buttock and does not involve the gluteal fold). The target EFP area must also be evident when the subject is standing, without the use of any manipulation (such as skin pinching or muscle contraction).

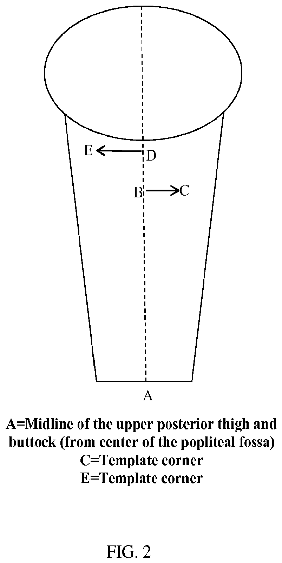

The investigator will then place the injection template over the target EFP area (while the subject remains standing) and position the template encircled 'X' over the center of the deepest depression (for example, the central dimple) in the target area. The investigator will use a surgical marker and mark the subject's skin at two corners of the template. The investigator will remove the template and record which two corners of the template were marked (e.g., upper right, lower right, upper left, lower left). The investigator will use a tape measure to measure as follows:
1. Identify the midline of the upper thigh and buttock starting from the center of the popliteal fossa (FIG. 2).
2. Identify the line that is perpendicular to the midline and goes through the first template corner mark (as depicted in FIG. 2, [Line BC]). Measure distance between Points B and C (record distance).
3. Measure the distance from Point A to Point B as depicted in FIG. 2 (record distance).
4. Repeat Steps 2 and 3 for measurement of the second template corner mark (FIG. 2, [Points A, D, and E]).
Each recorded measurement will be later used to relocate the 8 cm x 10 cm target EFP area before injection and before each efficacy evaluation time point.

On Day 1, after the injection site has been identified, marked (as described above), photographed, and the pre-injection efficacy evaluations have been completed, the subject will be positioned prone on the examination table so that the entire 8 cm x 10 cm target EFP area with the previously marked injection sites is visible and assessable to the investigator. The injection site area should be prepped with an appropriate antiseptic such as alcohol.

Using a previously prepared 5mL or 1 mL syringe (depending on cohort assignment) with a 30 gauge ¼ inch needle, the investigator will administer a dose of either 0.5 mL or 0.1 mL of Collagenase Clostridium Histolyticum into each of the 10 injection sites. At each of the 10 sites, study drug will be injected perpendicular to the subject's skin to a depth of ¼ inch (∼7 mm).

The investigator will administer study drug in a sequence that ensures each of the 10 sites receives a single 0.5 mL or 0.1 mL injection of Collagenase Clostridium Histolyticum. Following the tenth injection, the maximum permissible volume of study drug will have been administered (5 mL or 1 mL). Any injectate on the surface of an injection site following injection (suggestive of extravasation) will be noted and the associated injection location will be recorded. No local massage or palpation will be performed subsequent to injection of study drug.

The following primary endpoints will be evaluated at Day 90:
- Investigator global aesthetic improvement scale assessment of the target EFP area;
- Subject global aesthetic improvement scale assessment of the target EFP area; and
- A responder analysis based on proportion of subjects with a ≥ 30% improvement from baseline in surface contour standard deviation of the target EFP area based on 3-D digital photography.

While this invention has been particularly shown and described with references to preferred embodiments thereof, it will be understood by those skilled in the art that various changes in form and details may be made therein without departing from the scope of the invention encompassed by the appended claims.

## Claims

1. A cosmetic method of treating or reducing edematous fibrosclerotic panniculopathy (EFP) in a patient comprising the steps of: providing a pharmaceutical composition comprising a pharmaceutically acceptable carrier, collagenase I and collagenase II in a 1:1 mass ratio with a specific activity of 10,000 ABC units per 0.58 mg of collagenase, and administering by subdermal injection to said patient 5-200 ABC units per injection of the pharmaceutical composition to an area affected by EFP, wherein when multiple injections are administered, injection sites are about 1 to about 4 cm from one another.

2. The method of claim 1, wherein a plurality of subdermal injections are administered at a plurality of injection sites.

3. The method of claim 1, wherein the concentration of collagenase administered is between 50 to 2000 ABC units per milliliter.

4. The method of any one of claims 1 to 3, wherein each injection of collagenase is administered in a volume of about 0.5 ml or less.

5. The method of any one of claims 1 to 4 wherein the total dose of collagenase administered is between about 5 to about 2000 ABC units.

6. The method of any one of the preceding claims, wherein the dose of collagenase per injection is between 5 to 100 ABC units, preferably between about 5 to about 50 ABC units.

7. The method of any one of the preceding claims, wherein the collagenase is purified from *Clostridium histolyticum.*

8. The method of any one of the preceding claims, wherein the collagenase is a recombinant collagenase.

9. The method of any one of claims 1 to 4, wherein at least two injections, preferably at least five injections, more preferably at least ten injections of collagenase are administered to two, five or ten injection sites, respectively, within the affected area.

10. The method of any of the preceding claims, wherein the affected area of EFP has an area of 1 cm² to 200 cm².

11. The method of any one of claims 1 to 4, wherein plurality of subdermal injections of collagenase are administered and wherein the distance between injection sites is 2 to 3 cm.

12. The method of claim 9, wherein the area affected by EFP has a length of about 10 cm and width of about 8 cm.

13. The method of claim 11, wherein there is a distance of about 2.5 cm between injection sites.

14. The method of any one of claims 1 to 4, wherein each injection has a volume of about 0.1 ml.

15. The method of claim 5, wherein the total dose of collagenase administered to the affected area is between 5 to 1000 ABC units.

16. The method of any preceding claim, wherein each injection has a volume of about 0.2 ml or less.

## Patentansprüche

1. Kosmetisches Verfahren zum Behandeln oder Reduzieren einer ödematösen fibrosklerotischen Panniculopathie (EFP) bei einem Patienten, umfassend die Schritte: Bereitstellen einer pharmazeutischen Zusammensetzung, umfassend einen pharmazeutisch akzeptablen Träger, Kollagenase I und Kollagenase II in einem Massenverhältnis von 1:1 mit einer spezifischen Aktivität von 10000 ABC-Einheiten pro 0,58 mg Kollagenase und Verabreichen von 5 bis 200 ABC-Einheiten pro Injektion der pharmazeutischen Zusammensetzung durch subdermale Injektion an den Patienten in einem von EFP betroffenen Bereich, wobei die Injektionsstellen bei der Verabreichung von mehrfachen Injektionen etwa 1 bis etwa 4 cm voneinander beabstandet sind.

2. Verfahren nach Anspruch 1, wobei eine Vielzahl von subdermalen Injektionen an einer Vielzahl von Injektionsstellen verabreicht wird.

3. Verfahren nach Anspruch 1, wobei die Konzentration der verabreichten Kollagenase zwischen 50 und 2000 ABC-Einheiten pro Milliliter liegt.

4. Verfahren nach einem der Ansprüche 1 bis 3, wobei jede Injektion von Kollagenase in einem Volumen von etwa 0,5 ml oder weniger verabreicht wird.

5. Verfahren nach einem der Ansprüche 1 bis 4, wobei die verabreichte Kollagenase-Gesamtdosis zwischen etwa 5 und etwa 2000 ABC-Einheiten liegt.

6. Verfahren nach einem der vorhergehenden Ansprüche, wobei die Kollagenase-Dosis pro Injektion zwischen 5 und 100 ABC-Einheiten, vorzugsweise zwischen etwa 5 und etwa 50 ABC-Einheiten liegt.

7. Verfahren nach einem der vorangehenden Ansprüche, wobei die Kollagenase aus *Clostridium histolyticum* gereinigt wird.

8. Verfahren nach einem der vorhergehenden Ansprüche, wobei die Kollagenase eine rekombinante Kollagenase ist.

9. Verfahren nach einem der Ansprüche 1 bis 4, wobei mindestens zwei Injektionen, vorzugsweise mindestens fünf Injektionen, stärker bevorzugt mindestens zehn Injektionen von Kollagenase an zwei, fünf bzw. zehn Injektionsstellen innerhalb des betroffenen Bereichs verabreicht werden.

10. Verfahren nach einem der vorhergehenden Ansprüche, wobei der betroffene EFP-Bereich eine Fläche von 1 cm² bis 200 cm² hat.

11. Verfahren nach einem der Ansprüche 1 bis 4, wobei eine Vielzahl subdermaler Injektionen von Kollagenase verabreicht wird und wobei der Abstand zwischen den Injektionsstellen 2 bis 3 cm beträgt.

12. Verfahren nach Anspruch 9, wobei der von EFP betroffene Bereich eine Länge von etwa 10 cm und eine Breite von etwa 8 cm hat.

13. Verfahren nach Anspruch 11, wobei zwischen den Injektionsstellen ein Abstand von etwa 2,5 cm besteht.

14. Verfahren nach einem der Ansprüche 1 bis 4, wobei jede Injektion ein Volumen von etwa 0,1 ml hat.

15. Verfahren nach Anspruch 5, wobei die Kollagenase-Gesamtdosis, die an den betroffenen Bereich verabreicht wird, zwischen 5 und 1000 ABC-Einheiten liegt.

16. Verfahren nach einem vorhergehenden Anspruch, wobei jede Injektion ein Volumen von etwa 0,2 ml oder weniger hat.

## Revendications

1. Procédé cosmétique de traitement ou de réduction de la panniculopathie oedémato-fibro-sclérotique (EFP) chez un patient comprenant les étapes de : fourniture d'une composition pharmaceutique comprenant un véhicule pharmaceutiquement acceptable, de la collagénase I et de la collagénase II dans un rapport en masse 1:1 avec une activité spécifique de 10 000 unités ABC par 0,58 mg de collagénase, et administration par injection sous-dermique audit patient de 5 à 200 unités ABC par injection de la composition pharmaceutique à une zone affectée par EFP, dans lequel, lorsque des injections multiples sont administrées, les sites d'injection sont à environ 1 à environ 4 cm les uns des autres.

2. Procédé selon la revendication 1, dans lequel une pluralité d'injections sous-dermiques sont administrées à une pluralité de sites d'injection.

3. Procédé selon la revendication 1, dans lequel la concentration de collagénase administrée est comprise entre 50 et 2000 unités ABC par millilitre.

4. Procédé selon l'une quelconque des revendications 1 à 3, dans lequel chaque injection de collagénase est administrée dans un volume d'environ 0,5 ml ou moins.

5. Procédé selon l'une quelconque des revendications 1 à 4, dans lequel la dose totale de collagénase administrée est comprise entre environ 5 et environ 2000 unités ABC.

6. Procédé selon l'une quelconque des revendications précédentes, dans lequel la dose de collagénase par injection est comprise entre 5 et 100 unités ABC, de préférence entre environ 5 et environ 50 unités ABC.

7. Procédé selon l'une quelconque des revendications précédentes, dans lequel la collagénase est purifiée à partir de Clostridium histolyticum.

8. Procédé selon l'une quelconque des revendications précédentes, dans lequel la collagénase est une collagénase recombinante.

9. Procédé selon l'une quelconque des revendications 1 à 4, dans lequel au moins deux injections, de préférence au moins cinq injections, plus préférablement au moins dix injections de collagénase sont administrées à deux, cinq ou dix sites d'injection, respectivement, dans la zone affectée.

10. Procédé selon l'une quelconque des revendications précédentes, dans lequel la zone affectée d'EFP a une aire de 1 cm² à 200 cm².

11. Procédé selon l'une quelconque des revendications 1 à 4, dans lequel la pluralité d'injections sous-dermiques de collagénase sont administrées et dans lequel la distance entre les sites d'injection est de 2 à 3 cm.

12. Procédé selon la revendication 9, dans lequel la zone affectée par EFP a une longueur d'environ 10 cm et une largeur d'environ 8 cm.

13. Procédé selon la revendication 11, dans lequel la distance entre les sites d'injection est d'environ 2,5 cm.

14. Procédé selon l'une quelconque des revendications 1 à 4, dans lequel chaque injection a un volume d'environ 0,1 ml.

15. Procédé selon la revendication 5, dans lequel la dose totale de collagénase administrée à la zone affectée est comprise entre 5 et 1000 unités ABC.

16. Procédé selon l'une quelconque des revendications précédentes, dans lequel chaque injection a un volume d'environ 0,2 ml ou moins.
